# EUROPEAN PATENT APPLICATION

(11) **EP 3 395 349 A1**
(43) Date of publication of application: **31.10.2018**
(21) Application number: 16879010.3
(22) Date of filing: 22.12.2016
(51) Int. Cl.: A61K 35/15, A61K 35/14, A61K 35/16, A61K 47/42, A61P 35/00, A61P 37/04, A61P 43/00

(54) **NKT CELL-ACTIVATING PHARMACEUTICAL COMPOSITION, METHOD FOR PRODUCING SAME, AND METHOD FOR STORING ANTIGEN-PRESENTING CELL**

(30) Priority: 25.12.2015 JP 2015254533
(71) Applicant: Fujisoft Incorporated, Yokohama-shi, Kanagawa 231-8008 (JP); National University Corporation Chiba University, Chiba-shi, Chiba 267-8522 (JP)
(72) Inventor: MOTOHASHI, Shinichiro, Chiba-shi Chiba 260-8670 (JP); HARAI, Motohiro, Yokohama-shi Kanagawa 231-8008 (JP)
(74) Representative: Fuchs Patentanwälte Partnerschaft mbB
(86) International application number: PCT/JP2016/088561
(87) International publication number: WO 2017/111124

(57) **Abstract**

The present invention address the problem of providing an NKT cell-activating pharmaceutical composition having an improved storage period, a method for producing the pharmaceutical composition, and a method for storing an antigen-presenting cell. According to one embodiment, a pharmaceutical composition includes a dendritic cell as an active component, expressing a CDld molecule on a surface thereof, α-galactosylceramide being bound to the CD1d molecule, plasma at a concentration of 40 volume/volume % or higher to a total volume and a physiological solution containing the dendritic cell and the plasma.

## Description

### Technical Field

Embodiments described herein relate generally to an NKT cell activation pharmaceutical composition, a production method thereof and a method of storing an antigen presenting cell.

### Background Art

As an effective therapeutic method for malignant tumor or the like, an immunotherapy which activates natural killer T (NKT) cells inside the body of a subject is known. The NKT cells have antitumor activity. The NKT cells are known to be increased and activated by α-galactosylceramide (α-GalCer). To activate NKT cells, a method comprising administering to a subject a cell product which contains dendritic cells (antigen presenting cells) derived from the subject, presenting α-GalCer is used (Non Patent Literature 1).

Such a cell product has a short storable period of the antigen presenting cells contained therein. On the other hand, in some cases, it is desirable to delay the timing of use of the cell product depending on the conditions of the subject. However, once the preparation of the product is started, it is not possible to greatly change the day of the administration according to the conditions of the patient. Moreover, since the storable period is short, the product cannot be transported long way. Under these circumstances, in order to be able to administer the cell product while the survival ratio of the antigen presenting cells contained therein is still high, the cell product is cultured and prepared in the institution where it is administered to the subject, and then administered to the subject as soon as possible.

### Citation List

### Non Patent Literature

Non Patent Literature 1: Ishikawa E.et al. and Int. J. Cancer, 117, 265-273 (2005)

### Summary of Invention

### Technical Problem

One of embodiments described herein aims to provide an NKT cell activation pharmaceutical composition which has an improved storable period, a production method thereof, and a method of storing an antigen presenting cell.

### Means for Solving the Problem

The NKT cell activation pharmaceutical composition according to the embodiment contains dendritic cells as an active component, and plasma at a concentration of 40 volume/volume % or higher to the total volume of the composition. The dendritic cells express CD1d molecules on the surfaces thereof. To the CDld molecules, α-galactosylceramide is bound. The dendritic cells and the plasma are contained in a physiological solution.

### Advantages of Invention

According to the embodiment, it is possible to improve the storable period of the antigen presenting cells in the pharmaceutical composition for activating NKT cells.

### Brief Description of Drawings

FIG. 1 is a schematic diagram showing an example of an antigen presenting cell of an embodiment.
FIG. 2 is a flowchart illustrating an example of a method of producing a pharmaceutical composition of an embodiment.
FIG. 3 is a flowchart illustrating an example of a method of producing the pharmaceutical composition of the embodiment.
FIG. 4 is a flowchart illustrating an example of a method of storing an antigen presenting cell of an embodiment.
FIG. 5 is a diagram showing a result in Example 1 of the embodiment.
FIG. 6 is a graph showing a result of Example 7 of the embodiment.

### Mode for Carrying Out the Invention

Embodiments will be described below with reference to the accompanying drawings.

### 1. Pharmaceutical composition for activating NKT cell

An example of the pharmaceutical composition according to an embodiment contains dendritic cells as an activity component, plasma at a concentration of 40 volume/volume % or higher to the total volume of the composition, and a physiological solution containing the dendritic cell and the plasma. The dendritic cells express a CDld molecule on the surfaces, and α-galactosylceramide (to be referred to as "a-GalCer" hereinafter) is bound to the CD1d molecule. Such a dendritic cell will be called an antigen presenting cell hereinafter.

The antigen presenting cell presents α-GalCer through the CD1d molecule expressed on the dendritic cell derived from mononuclear cell. With such a structure, the antigen presenting cell activates an NKT cell directly by presenting α-GalCer as a ligand in the body of the subject to which the cell was administered. When α-GalCer binds to a receptor of an NKT cell, the NKT cell is activated. The activated NKT cell attacks tumor cells directly and at the same time induces the production of IFN-γ and the like. The IFN-γ produced activates the NK cells and CD8 T cells. These cells each indirectly attack malignant tumor cells. Moreover, the dendritic cells which support α-GalCer mature by stimulus of CD40L from the activated NKT cells. As a result, the CD8 T cells are activated. By such an action mechanism, the pharmaceutical composition of the embodiment exhibits an advantageous effect in the treatment of malignant tumors.

Examples of the malignant tumors are skin cancer, lung cancer, thyroid cancer, stomach cancer, esophagus cancer, bile duct cancer, colon cancer, rectal cancer, liver cancer, kidney cancer, pancreatic cancer, head and neck cancer, breast cancer, bladder cancer, ovary cancer, uterine cancer, prostatic cancer, testicular tumor, pediatric cancer, malignant lymphoma, myeloma, brain tumor, leukemia, tumors in bones and soft-parts, and any combination of these.

The subject may be, for example, any one of mammalians including rodents such as mice and rats, companion animals such as rabbits, dogs and cats, livestock including horses and cows and primates such as apes and humans.

Conventionally, such a method is generally employed that antigen presenting cells are suspended in an albumin-added physiological saline, and the resultant is administered to a subject. However, with such a suspension, it is very difficult to ensure a survival ratio of 45% or higher of the antigen presenting cells for over three days. According to the NKT cell activation pharmaceutical composition of the embodiment, the antigen presenting cells can be stored longer than by the conventional technique, for example, over about eight days after the production while keeping a survival ratio of 45% or higher. In this way, it is also possible to use up to 24 hours of the storable time for transportation from the place where the pharmaceutical composition is produced to the place where it is administered to the subject.

### 2. Component contained in the pharmaceutical composition for activating NKT cells

### (1) Antigen Presenting Cells

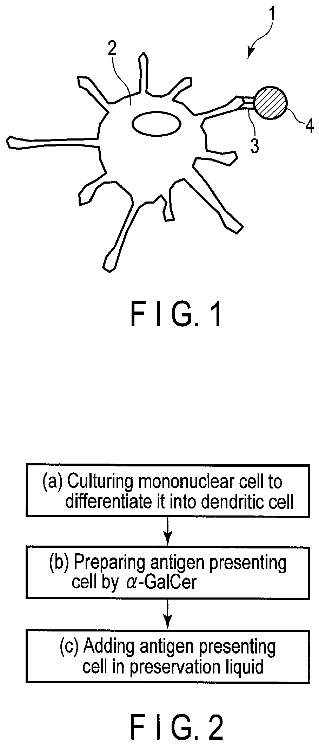

The pharmaceutical composition of the embodiment is a composition for activating NKT cells, and contains antigen presenting cells as an active component. As shown in FIG. 1, an antigen presenting cell 1 is a cell in which α-GalCer4 is bound to a CDld molecule 3 of a surface of a dendritic cell 2.

The dendritic cell may be that expressing a CDld molecule on its surface. It may be produced by differentiation in vitro or in vivo. A dendritic cell differentiated in vitro may be of the mononuclear cell origin. The mononuclear cell is, for example, the peripheral blood mononuclear cell. The dendritic cell differentiated in vivo may be, for example, any of myeloid dendritic cells, or dendritic cells derived from a tissue, or a combination of any of these extracted from the subject. For example, the antigen presenting cell may exist in the pharmaceutical composition at a concentration of 1.0 × 10⁷ to 2.5 × 10⁷ pieces/mL.

### (2) Physiological solution

In the pharmaceutical composition for activating NKT cells, the antigen presenting cells are contained in a physiological solution. The physiological solution is a carrier for maintaining the component contained in the pharmaceutical composition and carrying the component contained therein into an organism. The physiological solution may be, for examples, a physiological saline, a biological buffer, an electrolytic liquid, a carbohydrate liquid or the like. The physiological solution should preferably have a pH value of 3.5 to 8.0 and be non-toxic to organisms and non-irritant.

The physiological solution contains antigen presenting cells as an active component and plasma as a component for storing the antigen presenting cells. The concentration of the plasma is 40 volume/volume % or higher to the total volume. The plasma may be contained in the pharmaceutical composition by about 40 volume/volume % or more, or about 45 volume/volume % or more to the total volume, for example, 40 to 60 volume/volume %, 45 to 55 volume/volume % or 45 to 60 volume/volume %. With such a concentration of plasma in the physiological solution, the survival ratio of the antigen presenting cells is maintained high and the storable period is improved.

Thus, the physiological solution containing the plasma at a concentration 40 volume/volume % or higher is a carrier of the component contained in the pharmaceutical composition and it also serves as a preservation liquid which enable a long term storage of the antigen presenting cells. Hereinafter, the physiological solution containing the plasma at a concentration 40 volume/volume % or higher will be referred to as a "preservation liquid".

The plasma may be autologous plasma or non-autologous plasma. The autologous plasma is derived from the subject to which the pharmaceutical composition is to be administered. The non-autologous plasma may be derived from a mammalian other than the subject to which the pharmaceutical composition is to be administered.

### (3) Other components for preservation

The preservation liquid may further contain, as components for storing antigen presenting cells, a cytokine which transmits signals via a receptor containing a common γ chain (which will be referred to as "cytokine" hereinafter), and/or insulin. The pharmaceutical composition may further contain albumin as a component for stably maintaining the protein in the preservation liquid.

The characteristic feature of the cytokine is that the receptor contains a common γ chain (Common Gamma Chain, γc, CD132). The receptor may contain an α chain or a β chain or the like, in addition to the γ chain. The receptor may be, for example, IL-2 receptor, IL-7 receptor, IL-15 receptor or the like. The cytokine enables to maintain high survival ratio of antigen presenting cells for a long time. The cytokine may be, for example, IL-2, IL-7, IL-15 or the like. They may be used solely or in combination. The concentration of the cytokine in the pharmaceutical composition may be, for example, 1 to 1,000 JRU/mL.

Insulin is one of the growth factors, which enables to stably maintain the condition of antigen presenting cells. As long as the component has a growth factor of such characteristics, it is possible to replace the insulin by some other type of component to be used partially or in combination with insulin. The concentration of the insulin in the pharmaceutical composition may be 0.0142 to 0.142 U/mL.

The other type of component may be, for example, an insulin analogue which is varied in such a range which can stabilize and maintain the condition of antigen presenting cells.

The albumin may be human albumin when the pharmaceutical composition is to be administered to a human. When the pharmaceutical composition is to be administered to a mammal other than human, it may be selected according to the animal species of the mammal. The concentration of the albumin in the pharmaceutical composition may be 5% to 10%.

### (4) Other components

The pharmaceutical composition for activating NKT cells may contain, in addition to the components indicated in items (2) to (4) above, a solubilizer, a buffer, an isotonizing agent, a stabilizer or a soothing agent, or a combination of any of these, which does not affect the activity of any of the components.

The NKT cell activation pharmaceutical composition prepared as above may be used by administering it to the subject. The administration route may be, for example, intradermal, hypodermal, intramuscular, intraarterial, intravenous, into a tumor, mucosal, sub mucosal, or a combination of any of these. The dosage form may be, for example, injection or drip infusion.

The NKT cell activation pharmaceutical composition may be administered to the subject with the effective dose at one time. The effective dose may be selected according to the age, height, weight, sex, the degree of advance of the malignant tumor and the like, of the subject to which the NKT cell activation pharmaceutical composition is to be administered.

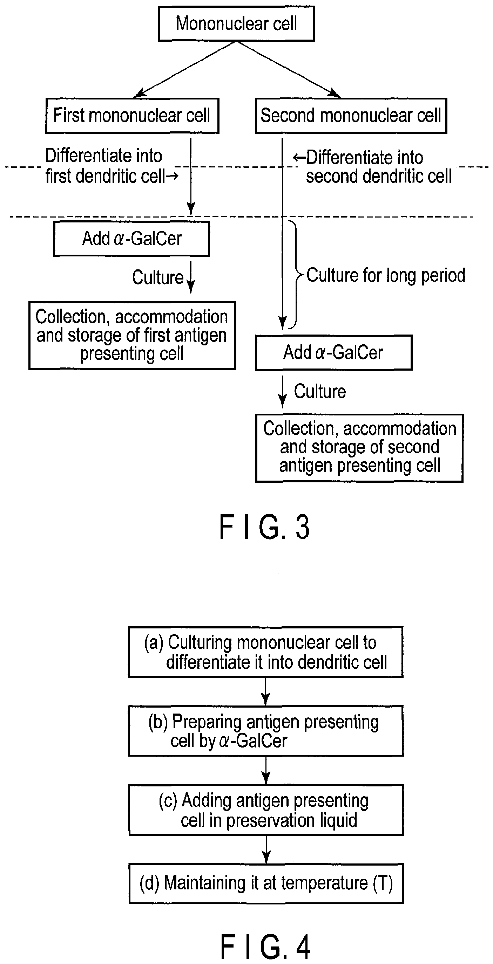

Moreover, when administering first and second NKT cell activation pharmaceutical compositions obtained by dividing a mononuclear cell into two and culturing them as shown in FIG. 4, it is preferable to administer the first NKT cell activation pharmaceutical composition to the subject, and then three to seven days later, administer the second NKT cell activation pharmaceutical composition.

### 3. Method of producing NKT cell activation pharmaceutical composition

According to an embodiment, a method of producing a pharmaceutical composition for activating NKT cells is provided. The production method may comprise the following steps (a) to (c) as shown in FIG. 2:
(a) differentiating a mononuclear cell to obtain a dendritic cell which expresses a CDld molecule on the surface;
(b) binding α-GalCer to the dendritic cell obtained in step (a) to obtain an antigen presenting cell; and
(c) adding the antigen presenting cell obtained in step (b) into a preservation liquid which contains the plasma at a concentration 40 volume/volume % or higher to the total volume, to obtain an NKT cell activation pharmaceutical composition.

Such a production method of an NKT cell activation pharmaceutical composition will now be further described.

### (a) Differentiation of mononuclear cell into dendritic cell which expresses CD1d molecule on the surface

The differentiation of a mononuclear cell into a dendritic cell which expresses a CDld molecule on the surface may be performed, for example, by culturing the mononuclear cell by a culture medium which contains a component to differentiate a mononuclear cell into a dendritic cell. The culture medium may contain, for example, the cytokine, a granulocyte macrophage colony-stimulating factor (GM-CSF) and plasma. The cytokine, GM-CSF and plasma may be contained at concentrations of, for example, about 100 JRU/mL, about 800 U/mL and about 2.5%, respectively, in the culture medium.

The basal medium of the culture medium may be a liquid medium which contains the above-described materials to be able to culture cells, it may be, for example, AIM-V (registered trademark) or RPMI-1640.

The culture medium may be a commercially available culture medium, for example, X-VIVO (registered trademark) (LONZA), Humankine G4 Dendritic Cell Generation Kit (of HumanZyme, Inc.), or Dendritic-cell Differentiation Culture Medium (of Takara Bio, Inc.).

When culturing by a culture medium as described above, the cell which has expressed a CDld molecule to the cell surface in the mononuclear cell can differentiate into dendritic cells about five to seven days after starting culture. The differentiation may be induced by a CD11c⁻CD3⁺ T cell contained in the mononuclear cell.

### (b) Preparation of antigen presenting cells

Antigen presenting cells may be obtained by, for example, adding α-GalCer to dendritic cells obtained by culturing the mononuclear cell for six to thirteen days by the culture medium, and then further culturing the cells for one to two days. Here, the addition of α-GalCer may be performed by adding a α-GalCer solution prepared to have an α-GalCer concentration of 100 to 200 ng/mL in the culture medium, to the culture medium. The α-GalCer solution may be prepared by, for example, dissolving α-GalCer in a solvent such as distilled water. The α-GalCer may be, for example, KRN7000 etc.

In a further embodiment, antigen presenting cells may be prepared by a method comprising the following steps shown in FIG. 3:
dividing a mononuclear cell and differentiating a first mononuclear cell into a first dendritic cell,
culturing a second mononuclear cell by a culture medium similar to that used for culturing the first mononuclear cell for a time period longer than that of a first peripheral blood cell, for example, ten to fourteen days to differentiate it into a second dendritic cell; and
adding α-GalCer to each of the first dendritic cell and the second dendritic cell and cultivating them for one to two days, thereby obtaining a first antigen presenting cell and a second antigen presenting cell.

The first mononuclear cell and the second mononuclear cell can differentiate respectively into dendritic cells at similar stages. And it may take for the second dendritic cell a long period to be cultured as a dendritic cell. The first antigen presenting cell and the second antigen presenting cell are each collected by a method, which will be described later in similar manners when culture of a respective predetermined period is finished and then suspended in separate preservation liquids, respectively. They may be accommodated and reserved in separate containers, respectively.

### (c) Mixing antigen presenting cells into preservation liquid and preparation of NKT cell activation pharmaceutical composition

The antigen presenting cells prepared as above may be collected when the culture of a predetermined period is finished. The antigen presenting cells may be collected, for example, by dispensing them into centrifugation pipe with a pipet, followed by centrifuging. The centrifuging should preferably be carried out at 1,200 to 1,500 rpm for 5 to 10 minutes at room temperature. To the antigen presenting cells thus collected, a human albumin-added physiological saline may be added to wash it. Thereafter, the number of the collected antigen presenting cells may be counted. The number of the cells should just be about 2 × 10⁹ to 5 × 10⁹. The number of the cells may be measured by any one of the well-known procedures.

The preservation liquid contains a physiological solution as a solvent such as a physiological saline, a physiological buffer, an electrolytic liquid or a carbohydrate liquid, and plasma contained at a concentration of 40 volume/volume % or higher to the total volume. The preservation liquid may be prepared by adding 40% or more of plasma by volume ratio to the physiological solution sterilized by filtration or autoclave sterilization. The plasma may be autologous plasma or non-autologous plasma.

The plasma usable here can be obtained by preparing it in the medical institution itself or the like or from commercially available types. In such a medical institution, the plasma may be extracted as follows, for example. That is, first, blood may be collected from a donor who provides plasma. The blood should preferably contain about 100 to 300 mL of plasma. Next, the mononuclear cells may be removed from the blood. It is preferable that at least 80 to 240 mL of plasma remains in the blood from which the mononuclear cells have been removed. The autologous plasma may be derived from the blood extracted from the subject to which the pharmaceutical composition is to be administered. The non-autologous plasma may be of blood extracted a mammal other than the subject to which the pharmaceutical composition is to be administered. The plasma may be extracted from the same blood as that from which the mononuclear cells have been removed, or from blood extracted separately. The plasma may be used in step (a) and/or step (c).

To the preservation liquid, the cytokine, insulin, albumin, or a combination of any of these may be added in addition to the plasma. The cytokine may be, for example, IL-2, IL-7, IL-15 or the like, etc., and IL-2 may be, for example, Immunace (registered trademark) (Shionogi & Co.). The insulin may be, for example, 100 units/mL of Humalin R (registered trademark). The albumin may be, for example, "Kenketsu (blood donation) Albumin" (KAKETSUKEN).

In a further embodiment, the preservation liquid prepared using non-autologous plasma may be prepared using a blood product. For example, the preservation liquid may be prepared by using a blood product in place of 40 volume/volume % or higher of the plasma derived from the subject and the albumin. The blood product may be a mixture of leukocyte component-removed blood and a blood preservation liquid. The blood preservation liquid may be, for example, ACD-A liquid or the like. The blood product may be, for example, fresh frozen- plasma-LR "Nisseki" or the like. The blood product may be used in accordance with the ABO blood type and the positivity/negativity in D (Rho) antigen of the subject to which the NKT cell activation pharmaceutical composition is to be administered. In that case, the preservation liquid may be prepared by diluting the blood product with a physiological solution so as to adjust that the concentration of the plasma to that indicated in Item 2. (2) set forth above. To the preservation liquid, cytokine and insulin may be added at respective concentrations as indicated in Item 2. (3).

To the preservation liquid prepared as described above, antigen presenting cells are added, and thus an NKT cell activation pharmaceutical composition is obtained.

The mononuclear cells used in the production method may be that prepared in a medical institution or the like, or a commercially available one. In such a medical institution, the mononuclear cells may be extracted, for example, as follows. First, blood may be collected from the donor who provides the peripheral blood cells. It is preferable that the collected blood contains about 2 × 10⁹ to 10 × 10⁷ mononuclear cells. Next, the mononuclear cells may be extracted from the collected blood. The extraction may be performed, for example, by diluting the blood with a physiological saline and subjecting the resultant to centrifuging, followed by extracting a layer where the mononuclear cells exist, from the blood subjected to centrifuging. The mononuclear cells should preferably be autologous mononuclear cells of the subject itself, but may be mononuclear cells obtained from a mammal other than the subject by a method similar to that described above. The steps (a) to (c) may be performed aseptically.

In the further embodiment, the pharmaceutical composition for activating NKT cells may be manufactured by the following steps:
(a) culturing a mononuclear cell in a culture medium containing cytokine, GM-CSF and a portion of the plasma, to differentiate it into a dendritic cell;
(b) binding α-GalCer to the dendritic cell obtained in step (a) to obtain an antigen presenting cell;
(c) collecting the antigen presenting cell;
(d) preparing a preservation liquid that contains cytokine, insulin and 40% or more by volume ratio of the plasma; and
(e) adding the antigen presenting cell obtained in step (d) into the preservation liquid which contains the plasma at a concentration of 40 volume/volume % or higher to the total volume, to obtain the NKT cell activation pharmaceutical composition.

The steps (a) to (e) may be similar to those of the production method described above.

### 4. Method of storing NKT cell activation pharmaceutical composition

A method of storing the NKT cell activation pharmaceutical composition will now be described. According to an embodiment, a method of storing the pharmaceutical composition for activating NKT cells is provided. As shown in FIG. 4, the storing method may comprise the following steps (a) to (d):
(a) differentiating a mononuclear cell, thereby obtaining a dendritic cell which expresses a CDld molecule;
(b) binding α-GalCer to the dendritic cell obtained in step (a), to obtain an antigen presenting cell;
(c) adding the antigen presenting cell obtained in step (b) into a preservation liquid which contains plasma at a concentration 40 volume/volume % or higher to a total volume, to obtain an NKT cell activation pharmaceutical composition; and
(d) maintaining the NKT cell activation pharmaceutical composition obtained in step (c) at a temperature (T) of: 0°C < T ≤ 8°C.

The steps (a) to (c), which cover until obtaining the NKT cell activation pharmaceutical composition, may be performed by the same method as those of the production method described above.

The NKT cell activation pharmaceutical composition prepared in the steps (a) to (c) may be contained in a container in an appropriate state for storage and transportation. The container may be, for example, an intravenous drip bag, ampule, syringe, a tube, a pack or the like. The composition may be contained aseptically in the container and the container may be sealed after containing the composition. Then, the container is brought into a cool box at a temperature (T) of: 0°C < T ≤ 8°C, and maintained therein. The temperature of the cool box should preferably be 2°C to 6°C, for example, may be about 4°C. The maintaining step may be performed in a hermetic condition. Thus, the antigen presenting cell in the NKT cell activation pharmaceutical composition can be reserved without gas exchange or exchange of the preservation liquid.

When an NKT cell activation pharmaceutical composition is produced and reserved as described above, the survival ratio of the antigen presenting cell is maintained at 45% or higher for about eight days. Of the eight days, within the first 24 hours, it can be transported. During the transportation, the NKT cell activation pharmaceutical composition may be maintained at 0°C to 8°C. The NKT cell activation pharmaceutical composition may be transported under a shaded environment.

In the further embodiment, the antigen presenting cell in the pharmaceutical composition for activating NKT cells may be stored by the following step:
(a) culturing a mononuclear cell in a culture medium containing cytokine, GM-CSF and a portion of the plasma, to differentiate it into a dendritic cells;
(b) binding α-GalCer to the dendritic cell obtained in step (a), to obtain an antigen presenting cell;
(c) collecting the antigen presenting cell;
(d) preparing a preservation liquid that contains cytokine, insulin and the plasma at a volume ratio of 40% or more;
(e) adding the antigen presenting cell obtained in step (d) into the preservation liquid which contains the plasma at a concentration of 40 volume/volume % or higher to a total volume, to obtain an NKT cell activation pharmaceutical composition; and
(f) keeping the NKT cell activation pharmaceutical composition obtained in step (c) still in a cool box at a temperature (T) of: 0°C < T ≤ 8°C.

The steps (a) to (f) may be similar to those of the preservation method described above.

### <Examples>

Examples of the production method of the NKT cell activation pharmaceutical composition and the storing method for the antigen presenting cell described in the embodiments will be provided.

### Example 1: Characteristic evaluation of peripheral blood mononuclear cell cultured with IL-2/GM-CSF

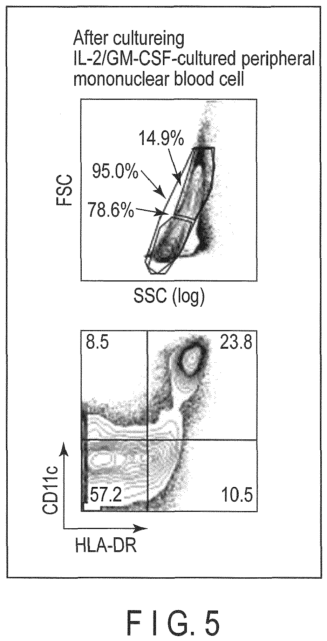

Peripheral blood mononuclear cells extracted from respective subject HV1 and HV2 were subjected to density gradient centrifugation, and washed once with PBS, then washed twice with a 3%-thermal inactivating FBS-added PBS or RPMI-1640. The washed peripheral blood mononuclear cells were cultured for seven days with RPMI-1640 which contains 100 JRU/mL of rhIL-2 (Imunace (registered trademark) of Shionogi, Inc.) and 800 U/mL of rhGM-CSF (NCPC Genetech Biotechnology Development Company), to which 10% of FBS, 0.01-m of M2-ME and 50 U/mL of penicillin-streptomycin were added. The thus obtained peripheral blood mononuclear cells cultured with IL-2/GM-CSF were cultured in the presence of 100 JRU/mL of IL-2 and 800 U/mL of GM-CSF. The resultants were subjected to analysis by flow cytometory (FCM) to measure the size of cells and detect the expressions of CD11c and HLA-DR. SSC/FSC plots of an IL-2/GM-CSF-cultured peripheral blood mononuclear cell before and after the culture is shown in an upper section of FIG. 5 part A, and an HLA-DR/CD11c plot of the cell is shown in an lower section of FIG. 5 part A. The peripheral blood mononuclear cell contained 15% of SSC^{high}FSC^{high} cell, which further contained 23.8% of a dendritic cell (HLA-DR⁺CD11c⁺ cell). Moreover, with the FCM using anti-CD11c-PE and anti-CD3-Cy (Pharmingen), it was clear that the peripheral blood mononuclear cell also contained a CD11c⁻CD3⁺ cell.

### Example 2: Evaluation of survival ratio of antigen presenting cell stored with preservation liquid that does not contain IL-2, insulin or autologous plasma

Blood was collected from a healthy 31 year-old male person and a healthy 34 year-old male person, and peripheral blood mononuclear cells were extracted respectively therefrom. The extracted cells were cultured with 100 JRU/mL of IL-2 and 800 U/mL of rhGM-CSF for six days as well as thirteen days, and then α-GalCer (100 ng/mL, KRN7000) was added. The resultants were cultured under conditions of 37°C and 5%-CO₂ for one day. The α-GalCer was prepared by the method discussed by Kawano et al. in Science, 1997. Thus, the antigen presenting cells cultured for a total of seven days (seven-day cultured cells) and the antigen presenting cell cultured for a total of fourteen days (14-day cultured cells) were prepared.

The antigen presenting cells of both types were partially collected and subjected to dyeing with trypan-blue, and the number of cells was counted. It was confirmed here that there were 90% or more of viable cells. The remaining cells were collected and dispensed into nine tubes each containing a physiological saline containing 25 mg/mL of albumin, and then suspended to obtain the suspensions containing 1.5 × 10⁶/100 µL of cells. These tubes were stored under a total of nine combinations of conditions, that is, they were let stand still at temperatures of 4°C, 10°C or 22°C for 24 hours, 48 hours or 72 hours, and the survival ratio of the cells was calculated for each case. The average value of three specimens was calculated.

The results are shown in Table 1. In both of the seven-day cultured cells and fourteen-day cultured cells, the survival ratio of 45% or higher was maintained even after 72 hours under conditions of 10°C and 22°C, but not under the condition of 4°C. Thus, it was made clear that the cells cannot be reserved stably for more than three days with a preservation liquid that does not contain IL-2, an insulin and autologous plasma.

**Table 1**

| | 7-day cultured cell (%) | | | 14-day cultured cell (%) | | |
|---|---|---|---|---|---|---|
| | Day 1 | Day 2 | Day 3 | Day 1 | Day 2 | Day 3 |
| 4°C | 100.6 | 80.5 | 44.1 | 64.2 | 42.7 | 32.7 |
| 10°C | 91.7 | 55.6 | 53.9 | 71.5 | 55.7 | 46.9 |
| 22°C | 86.5 | 68.8 | 49.7 | 82.2 | 60.5 | 53.4 |

### Example 3: Evaluation of survival ratio of antigen presenting cells transported and reserved preservation liquid not containing IL-2, insulin and autologous plasma

With a method similar to that of Example 2, seven-day cultured cells and fourteen-day cultured cells were prepared from a healthy 35 year-old male person. Both types of the antigen presenting cells were collected and suspended in respective physiological salines containing 25 mg/mL of albumin, to obtain the suspensions containing 1.5 × 10⁸/100 µL of cells respectively. They were divided into a half and dispensed into 15 mL-conical tubes, respectively. After checking those cells to have a survival ratio of 90% or more, they were packed in boxes of a low temperature (2°C to 8°C) and a room temperature (15°C to 25°C) together with a temperature data logger, and transported under both a low temperature and room temperature conditions for 16 hours. They were transported by a deliverance service company (Saroute Co., Ltd.) by vehicles from Chiba University (1-8-1, Inohana, Chuo-ku, Chiba-shi, Chiba-ken) to Safety Research Institute for Chemical Compounds Co., Ltd. (363-24, Shinei, Kiyota-ku, Sapporo-shi, Hokkaido). After that, they were stored at the same temperatures as the respective temperatures during the transportation, and then subjected to viable cell count after 48 hours and 72 hours. The temperatures during the transportation were 5.2°C to 5.8°C (low temperature) and 19.4°C to 22.2°C (room temperature) for the seven-day cultured cells and 4.4°C to 5.4°C (low temperature) and 19.6°C to 21.9°C (room temperature) for the fourteen-day cultured cells.

The results indicate that 45% or more of the survival ratio until after 72 hours is achievable only in the seven-day cultured cells under the low-temperature condition (Table 2). Therefore, it was made clear that the storage temperature should preferably be 2°C to 8°C.

**Table 2**

| | | 0H | Just after start of transportation | 48 h | 72 h |
|---|---|---|---|---|---|
| 7-day cultured cells | Low temp | 1.50(100%) | 1.44(96%) | 1.01(67%) | 0.96(62%) |
| | Room temp | 1.50(100%) | 1.61(107%) | 0.29(39%) | 0.29(20%) |
| 14-day cultured cells | Low temp | 1.50 (100%) | 1.26(84%) | 0.56(61%) | 0.56(37%) |
| | Room temp | 1.50(100%) | 1.20(80%) | 0.58(39%) | 0.59(39%) |

### Example 4: Evaluation of survival ratio of antigen presenting cells stored with preservation liquid of the embodiment

With a method similar to that of Example 2, seven-day cultured cells and fourteen-day cultured cells were prepared from a healthy 47 year-old male person. The cells were suspended in preservation liquids of a total of ten types of conditions, that is, five high IL-2 and insulin concentration conditions (High) in which the albumin was contained at 25 mg/mL, IL-2 at 100 U/mL, insulin at 5 µg/mL and autologous plasma at 10, 20, 30, 40 and 50%, and five low IL-2 and insulin concentration conditions (Low) in which the albumin was contained at 25 mg/mL, IL-2 at 10 U/mL, insulin at 0.5 µg/mL and autologous plasma at 10, 20, 30, 40 and 50%. The albumin used here was -Kenketsu (blood donation) albumin 25% intravenous injection 5g/50mL "Benesis" (Mitsubishi Tanabe Pharma Corp.), IL-2 was Immunace 35 (registered trademark) (Shionogi & Co.). The insulin was Humalin R (registered trademark) (Eli Lilly Japan, Inc.). They were stored at 4°C and the survival ratio of the cells was measured after 96 hours, 144 hours, 240 hours and 336 hours.

The results are shown in Table 3. It was made clear that the cells can be stored at a survival ratio of 45% for six days with the preservation liquid containing IL-2, insulin and 40% or higher of plasma. Moreover, as the concentrations of IL-2 and insulin are higher, the ratio of the viable cell is higher, but if the concentration of plasma is 40% or higher, the cells can be stored for six days.

**Table 3**

| | IL-2 (U/ml) | Insulin (µg/ml) | Autologous plasma (%) | 7-day cultured cells (%) | | | | 14-day cultured cells (%) | | | |
|---|---|---|---|---|---|---|---|---|---|---|---|
| | | | | 4 days | 6 days | 10 days | 14 days | 4 days | 6 days | 10 days | 14 days |
| High | 100 | 5 (0.0005%) | 50 | 71.5 | 63.8 | 22.3 | 25.5 | 69.0 | 62.0 | 33.4 | 29.4 |
| | | | 40 | 76.8 | 62.3 | 32.3 | 28.3 | 63.2 | 55.2 | 32.7 | 25.9 |
| | | | 30 | 75.5 | 57.8 | 34.6 | 26.8 | 58.7 | 43.5 | 33.6 | 36.0 |
| | | | 20 | 76.5 | 59.5 | 39.5 | 29.3 | 56.7 | 43.3 | 24.0 | 28.2 |
| | | | 10 | 69.0 | 54.8 | 32.9 | 30.8 | 41.8 | 37.7 | 24.4 | 26.3 |
| Low | 10 | 0.5 (0.00005%) | 50 | 72.7 | 59.0 | 22.8 | 22.1 | 65.0 | 51.5 | 22.2 | 29.9 |
| | | | 40 | 67.2 | 69.3 | 29.3 | 26.1 | 55.0 | 50.5 | 19.5 | 29.9 |
| | | | 30 | 68.0 | 54.3 | 25.0 | 33.4 | 52.8 | 50.8 | 24.2 | 32.7 |
| | | | 20 | 62.8 | 52.5 | 35.3 | 29.3 | 49.0 | 43.5 | 28.5 | 26.4 |
| | | | 10 | 58.5 | 57.7 | 33.0 | 24.7 | 41.3 | 38.0 | 18.9 | 28.0 |

### Example 5: Evaluation of survival ratio of antigen presenting cells stored with preservation liquid of the embodiment

With a method similar to that of Example 2, seven-day cultured cells were prepared from a healthy 47 year-old male person, and seven day cultured cells and fourteen-day cultured cells were prepared from a healthy 35 year-old male person. The antigen presenting cells were suspended in preservation liquids of a total of four types of conditions, that is, one high IL-2 and insulin concentration condition (High) in which the albumin was contained at 25 mg/mL, IL-2 at 100 U/mL, insulin at 5 µg/mL and autologous plasma at 50%, and three low IL-2 and insulin concentration conditions (Low) in which the albumin was contained at 25 mg/mL, IL-2 at 10 U/mL, insulin at 0.5 µg/mL and autologous plasma at 40, 45 and 50%. The albumin, IL-2 and insulin used here were same as those products of Example 4. These cells were stored under conditions of at 4°C and for 96 hours, 144 hours and 192 hours, and thereafter the survival ratio of the cells was measured in each case.

The results for the 47 year-old male are shown in Table 4 and those for the 35 year-old male are shown in Table 5. Under the test conditions for the seven-day cultured cells of the 47 year-old male, the eight-days survival ratio was maintained at 45% or higher when the IL-2 and insulin concentrations are low and the plasma is contained at 40%. In the case of the 35 year-old male, the eight-day survival ratio was maintained at 45% or higher when the plasma is contained at 40%. The fourteen-day cultured cells were maintained for six days on the conditions that the IL-2 and insulin concentrations are high and the plasma is contained at 50%. Thus, it was made clear that with the preservation liquid of the embodiment, the seven-day cultured cells can be stored for a maximum of eight days, and the fourteen-day cultured cells can be stored for a maximum of six days.

**Table 4**

| | IL-2 (U/ml) | Insulin (µg/ml) | Autologous plasma (%) | 7-day cultured cells (%) | | |
|---|---|---|---|---|---|---|
| | | | | 4 days | 6 days | 8 days |
| High | 100 | 5 (0.0005%) | 50 | 68.1 | 58.8 | 38.3 |
| Low | 10 | 0.5 (0.00005%) | 50 | 71.0 | 59.8 | 34.6 |
| | | | 45 | 74.3 | 61.6 | 41.9 |
| | | | 40 | 65.4 | 57.6 | 45.8 |

**Table 5**

| | IL-2 (U/ml) | Insulin (µg/ml) | Autologous plasma (%) | 7-day cultured cells (%) | | | 14-day cultured cells (%) | | |
|---|---|---|---|---|---|---|---|---|---|
| | | | | 4 days | 6 days | 8 days | 4 days | 6 days | 8 days |
| High | 100 | 5 (0.0005%) | 50 | 88.3 | 72.7 | 55.5 | 61.7 | 50.2 | 27.0 |
| Low | 10 | 0.5 (0.00005%) | 50 | 88.0 | 75.0 | 49.2 | 61.7 | 39.7 | 25.2 |
| | | | 45 | 82.8 | 60.0 | 48.5 | 60.5 | 40.8 | 23.8 |
| | | | 40 | 77.8 | 64.7 | 39.0 | 63.5 | 33.8 | 18.8 |

### Example 6: Evaluation of survival ratio of antigen presenting cells derived from the blood, transported or not transported in preservation liquid of the embodiment

Blood was collected extracted from a healthy 35 year-old male person, and seven-day cultured cells and fourteen-day cultured cells were prepared from a portion of the blood by a method same as that of Example 2 in Chiba University. The remainder of the blood was transported to the Fuji Soft, Inc., Regeneration Medicine Research Division (2-19-7, Kotobashi, Sumida-ku, Tokyo), and the seven-day and fourteen-day cultured cells were prepared in a same method. For the transportation, the blood was dispensed into tubes and packed in boxes of 15°C to 25°C together with a temperature data logger. The transportation temperature was 15°C to 25°C. At each of the above-described locations, the respective antigen presenting cells were suspended in preservation liquids of a total of four types of conditions, that is, one high IL-2 and insulin concentration condition (High) in which the albumin was contained at 25 mg/mL, IL-2 at 100 U/mL, insulin at 5 µg/mL and autologous plasma at 50%, and three low IL-2 and insulin concentration conditions (Low) in which the albumin was contained at 25 mg/mL, IL-2 at 10 U/mL, insulin at 0.5 µg/mL and autologous plasma at 40, 45 and 50%. The albumin, IL-2 and insulin used here were same as those products of Example 4. These cells were stored at 4°C for 4 days, and then they were packed in respective boxes of 2°C to 8°C together with a temperature data logger. After that, they were transported by a deliverance service company (Saroute Co., Ltd.) by vehicles from Chiba University or the Fuji Soft, Inc., Regeneration Medicine Research Division to Safety Research Institute for Chemical Compounds Co., Ltd. The transportation time was about 24 hours and the transportation temperature was 2°C to 8°C. After that, they were subjected to viable cell count after 24 hours, 48 hours and 72 hours.

The results are shown in Table 6. Except for the case where the fourteen-day cultured cells collected from the blood, which were not transported and stored in preservation liquid containing IL-2 and insulin at high concentrations and 50% of plasma, a survival ratio of 45% or higher was maintained for 72 hours after the transportation. Thus, it was made clear that even if the cells are transported for 24 hours within the storable period, the cells can be stored for a maximum of eight days.

**Table 6**

| | Blood transport | IL-2 (U/ml) | Insulin (µg/ml) | Plasma (%) | 7-day cultured cells (%) | | | 14-day cultured cells (%) | | |
|---|---|---|---|---|---|---|---|---|---|---|
| | | | | | 24 h | 48 h | 72 h | 24 h | 48 h | 72 h |
| High | Not included | 100 | 5 | 50 | 99.9 | 70.5 | 80.5 | 67.9 | 49.2 | - |
| Low | | 10 | 0.5 | 50 | 104.2 | 90.5 | 87.2 | 67.7 | 70.1 | 57 |
| | | | | 45 | 82.9 | 85.4 | 78.7 | 74.3 | 61.7 | 47.9 |
| | | | | 40 | 88.4 | 74.3 | 68.1 | 68.7 | 62.3 | 50.9 |
| High | Included | 100 | 5 | 50 | 76.9 | 76.9 | 62 | 82.2 | 78.1 | 54.5 |
| Low | | 10 | 0.5 | 50 | 96.3 | 81.3 | 66.9 | 84.1 | 82.7 | 73.7 |
| | | | | 45 | 83.0 | 79.2 | 61.2 | 71.9 | 73.4 | 53.5 |
| | | | | 40 | 81.5 | 67.7 | 56.4 | 74.9 | 74.1 | 60.9 |

| | | | | | | | | | | |
|---|---|---|---|---|---|---|---|---|---|---|
| "-" indicates no data available | | | | | | | | | | |

### Example 7: Test on NKT cell activation efficiency of antigen presenting cells stored with preservation liquid of the embodiment

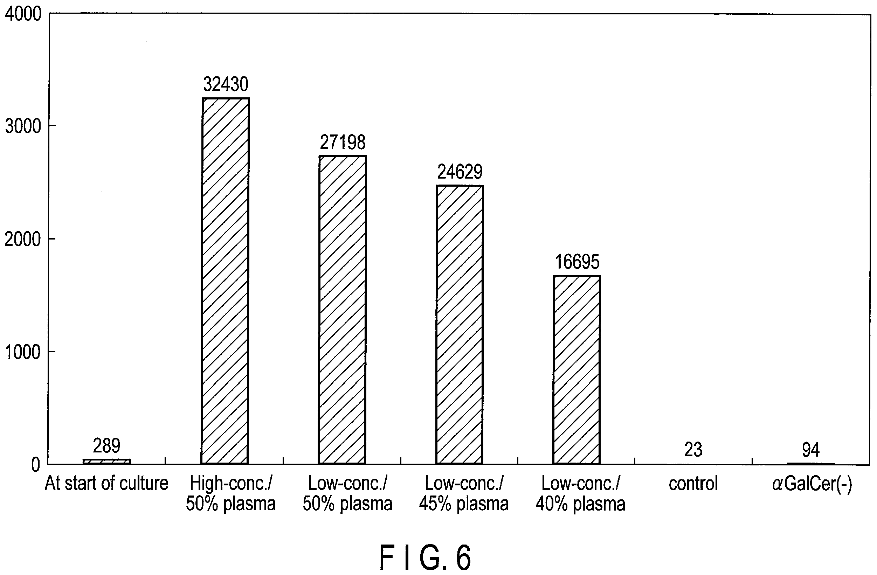

The seven-day cultured cells of the 35 years-old male prepared in Example 6 were suspended in a total of five types of conditions of preservation liquids (High and Low of Example 6, and one containing only albumin (25 mg/mL). As a control, the cells cultured for seven days without adding α-GalCer to the peripheral blood mononuclear cell derived from the same subject, were suspended in the preservation liquid (High). There samples were stored at 4°C for four days, and thereafter the NKT cells were annihilated by irradiation of radiation. Peripheral blood mononuclear cells were newly collected from the same subject, and the activation NKT cells were counted in number by detecting Vα24 and Vβ11 using FACS (FIG. 6, "At the start of culturing"). Then, the cultured cells subjected to the irradiation of radiation and the new peripheral blood mononuclear cells were co-cultured for seven days, and the cell number of the activation NKT cells was measured in each case.

The results are shown in FIG. 6. Under any conditions, when α-GalCer was added, the number of activation NKT cells was greater than the case where it was not added. Thus, it was made clear that the cells stored with the preservation liquid of the embodiment can activate NKT cells.

From Examples 2 to 7, it is now clear that the pharmaceutical compositions of the embodiment can store the antigen presenting cells at a survival ratio of 45% or more for a maximum of eight days, and within a transportation time of 24 hours, a survival ratio of 45% or more can be maintained thereafter and also the pharmaceutical composition can activate NKT cells.

### Reference Signs List

1 ... Antigen presenting cell; 2 ... Dendritic cell; 3 ... CDld molecule; 4 ... α-GalCer

## Claims

1. A pharmaceutical composition comprising:
a dendritic cell as an active component, wherein the dendritic cell expresses a CD1d molecule on a surface thereof, and α-galactosylceramide is bound to the CD1d molecule;
plasma at a concentration of 40 volume/volume % or higher to a total volume; and
a physiological solution containing the dendritic cell and the plasma.

2. The composition of Claim 1, wherein
the concentration of the plasma is 40 to 60 volume/volume %.

3. The composition of Claim 1, wherein
the concentration of the plasma is 45 to 55 volume/volume % or higher.

4. The pharmaceutical composition of Claim 1, wherein
the concentration of the plasma is 45 volume/volume % or higher.

5. The composition of any one of Claims 1 to 4, further comprising a cytokine which transmits a signal through a receptor containing a common γ chain.

6. The composition of Claim 1, further comprising an insulin or an insulin analogue.

7. The composition of Claim 1, further comprising albumin.

8. The composition of Claim 1, wherein
the plasma is autologous plasma.

9. The composition of Claim 1, wherein
the plasma is non-autologous plasma.

10. A method of producing a pharmaceutical composition for activating an NKT cell, the method comprising:
(a) differentiating a mononuclear cell to obtain a dendritic cell expressing a CDld molecule;
(b) binding α-galactosylceramide to the dendritic cell obtained in the above (a) to obtain an antigen presenting cell; and
(c) adding the antigen presenting cell obtained in the above (b) into a preservation liquid which contains plasma at a concentration of 40 volume/volume % or higher to a total volume to obtain a NKT cell activation pharmaceutical composition.

11. A method of storing an antigen presenting cell, the method comprising:
(a) differentiating a mononuclear cell to obtain a dendritic cell expressing a CDld molecule;
(b) binding α-galactosylceramide to the dendritic cell obtained in the above (a) to obtain an antigen presenting cell; and
(c) adding the antigen presenting cell obtained in the above (b) into a preservation liquid which contains plasma at a concentration of 40 volume/volume % or higher to a total volume to obtain an NKT cell activation pharmaceutical composition; and
(d) maintaining the NKT cell activation pharmaceutical composition obtained in the above (c) at a temperature (T) of 0°C < T ≤ 8°C.

12. The method of Claim 11, wherein
the maintaining in the above (d) is carried out during any period within eight days.

13. The method of Claim 11 or 12, wherein
the maintaining in the above (d) is carried out under a hermetic condition.
